# EUROPEAN PATENT SPECIFICATION

(11) **EP 3 411 330 B1**
(45) Date of publication and mention of the grant of the patent: **08.04.2026**
(21) Application number: 17748361.7
(22) Date of filing: 06.02.2017
(51) Int. Cl.: A61K 9/51, A61K 9/00, A61K 47/06, A61K 49/00, C01B 32/15, B82Y 5/00, A61P 3/10, A61P 19/00, A61P 25/28, A61P 43/00

(54) **CARBON DOTS FOR DIAGNOSTIC ANALYSIS AND DRUG DELIVERY**
KOHLENSTOFFQUANTENPUNKTE ZUR DIAGNOSTISCHEN ANALYSE UND WIRKSTOFFFREISETZUNG
POINTS DE CARBONE POUR UNE ANALYSE DIAGNOSTIQUE ET L'ADMINISTRATION DE MÉDICAMENTS

(30) Priority: 05.02.2016 US 201662292026 P
(43) Date of publication of application: 12.12.2018
(73) Proprietor: University of Miami, Miami, FL 33136 (US)
(72) Inventor: LI, Shanghao, Miami FL 33143 (US); LEBLANC, Roger M., Key Biscayne FL 33149 (US); SKROMNE, Isaac, Pinecrest FL 33156 (US); PENG, Zhili, Miami, FL 33136 (US)
(74) Representative: Inspicos P/S
(86) International application number: PCT/US2017/016743
(87) International publication number: WO 2017/136846

(56) References cited:
- WO-A1-2014/179708
- WO-A1-2016/053411
- WO-A2-2016/118214
- KR-A- 20160 003 488
- US-A1- 2013 156 856
- US-A1- 2016 039 678
- LV QING ET AL: "Characteristics of sequential targeting of brain glioma for transferrin-modified cisplatin liposome", INTERNATIONAL JOURNAL OF PHARMACEUTICS, ELSEVIER, AMSTERDAM, NL, vol. 444, no. 1, 21 January 2013 (2013-01-21), pages 1 - 9, XP028984482, ISSN: 0378-5173, DOI: 10.1016/J.IJPHARM.2013.01.025
- SHADABUL HAQUE ET AL: "Nanostructure-based drug delivery systems for brain targeting", JOURNAL DRUG DEVELOPMENT AND INDUSTRIAL PHARMACY, vol. 38, no. 4, 28 September 2011 (2011-09-28), US, pages 387 - 411, XP055537803, ISSN: 0363-9045, DOI: 10.3109/03639045.2011.608191
- LI JINGYAN ET AL: "PLA/PLGA nanoparticles for delivery of drugs across the blood-brain barrier", NANOTECHNOLOGY REVIEWS, vol. 2, no. 3, 5 March 2013 (2013-03-05), DE, pages 241 - 257, XP055849628, ISSN: 2191-9089, DOI: 10.1515/ntrev-2012-0084
- SHANGHAO LI ET AL: ""Dark" carbon dots specifically "light-up" calcified zebrafish bones", JOURNAL OF MATERIALS CHEMISTRY B, vol. 4, no. 46, 10 October 2016 (2016-10-10), GB, pages 7398 - 7405, XP055624997, ISSN: 2050-750X, DOI: 10.1039/C6TB02241C
- LI SHANGHAO ET AL: "Crossing the blood-brain-barrier with transferrin conjugated carbon dots: A zebrafish model study", COLLOIDS AND SURFACES. B, BIOINTERFACES, ELSEVIER, AMSTERDAM, NL, vol. 145, 5 May 2016 (2016-05-05), pages 251 - 256, XP029640046, ISSN: 0927-7765, DOI: 10.1016/J.COLSURFB.2016.05.007
- SHANGHAO LI ET AL: "Crossing the blood-brain-barrier with transferrin conjugated carbon dots: A zebrafish model study - Suppplementary data", COLLOIDS AND SURFACES. B, BIOINTERFACES, vol. 145, 5 May 2016 (2016-05-05), NL, pages 251 - 256, XP055625599, ISSN: 0927-7765, DOI: 10.1016/j.colsurfb.2016.05.007
- HUIYANG LIU ET AL: "A multifunctional ribonuclease A-conjugated carbon dot cluster nanosystem for synchronous cancer imaging and therapy", NANOSCALE RESEARCH LETTERS, vol. 9, no. 1, 15 August 2014 (2014-08-15), pages 397, XP055625037, ISSN: 1556-276X, DOI: 10.1186/1556-276X-9-397
- SONGEUN BEACK ET AL: "Photodynamic therapy of melanoma skin cancer using carbon dot - chlorin e6 - hyaluronate conjugate", ACTA BIOMATERIALIA, vol. 26, 1 October 2015 (2015-10-01), AMSTERDAM, NL, pages 295 - 305, XP055374179, ISSN: 1742-7061, DOI: 10.1016/j.actbio.2015.08.027
- YAN-FEI KANG ET AL: "Carbon Quantum Dots for Zebrafish Fluorescence Imaging", SCIENTIFIC REPORTS, vol. 5, no. 1, 2 July 2015 (2015-07-02), XP055625001, DOI: 10.1038/srep11835
- LI, S ET AL.: "Nontoxic Carbon Dots Potently Inhibit Human Insulin Fibrillation", CHEM. MATER., vol. 27, no. 5, 2015, pages 1764 - 1771, XP055405028
- JANG, M ET AL.: "Is the Chain of Oxidation and Reduction Process Reversible in Luminescent Graphene Quantum Dots?", SMALL, vol. 11, no. 31, 2015, pages 3773 - 3781, XP055405031
- WU, M ET AL.: "Preparation of functionalized water-soluble photoluminescent carbon quantum dots from petroleum coke", CARBON, vol. 78, 2014, pages 480 - 489, XP029043397
- SUN , H ET AL.: "Improvement of Photoluminescence of Graphene Quantum Dots with a Biocompatible Photochemical Reduction Pathway and Its Bioimaging Application", APPL. MATER. INTERFACES, vol. 5, 2013, pages 1174 - 1179, XP055136806

## Description

### Field of the Invention

The present disclosure relates to the formation of carbon dots and, more particularly, to the formation of carbon dots and their use for drug delivery.

### Background

The background description provided herein is for the purpose of generally presenting the context of the disclosure. Work of the presently named inventors, to the extent it is described in this background section or elsewhere herein, as well as aspects of the description that may not otherwise qualify as prior art at the time of filing, are neither expressly nor impliedly admitted as prior art against the present disclosure.

Carbon dots are quantum sized carbon nanoparticles which have recently emerged as benign nanoparticles with potential to replace heavy metal containing toxic quantum dots. The potential biological application of carbon dots has attracted great attention because of their unique properties, such as excitation wavelength dependent photoluminescence, excellent biocompatibility, low cytotoxicity, and optical stability.

Carbon dots can be prepared by a "top-down" or "bottom-up" approach, typically achieved by chemical, electrochemical, or physical techniques. "Top-down" synthetic routes refer to breaking down larger carbon structures such as graphite, carbon nanotubes, and nanodiamonds into carbon dots using laser ablation, arc discharge, and electrochemical techniques. In contrast, "Bottom-up" synthetic routes involve synthesizing carbon dots from small precursors such as carbohydrates, citrate, and polymer-silica nanocomposites through hydrothermal/solvothermal treatment, supported synthetic, and microwave synthetic routes.

Carbon dots can easily cross cellular membranes and, therefore, have potential applications in bioimaging and theranostics. However, for any practical application in biological systems, carbon dots will inevitably contact with peptides and proteins and may change their conformation. Known nanoparticles such as carbon nanotubes, cerium oxide nanoparticles, titanium dioxide nanoparticles, and gold nanoparticles have been found to promote fibrillation of proteins and peptides, resulting in changes to the protein/peptide structure.

Further, carbon dots have potential as therapeutic agents to treat neurodegenerative diseases inside the central nervous system (CNS). However, drug delivery to the CNS in biological systems remains a major medical challenge due to the presence of a highly selective permeability barrier, the blood-brain barrier (BBB).

Binding of carbon dots to live bone would be advantageous to provide a versatile drug delivery system. However, as demonstrated in "Functionalized carbon dots enable simultaneous bone crack detection and drug deposition," J. Mater. Chem. B 2014, 2, 8626-8632 and "In vitro detection of calcium in bone by modified carbon dots," Analyst 2013, 138, 7107-7111, carbon dots prepared according to published protocols only show bone-binding activity when conjugated to glutamic acid ( a calcium-binding molecule), and only in extracted bones, and never in live animals. Further, in order to load carbon dots with drugs, the surface of the carbon dots may need to be modified and such modifications may affect the binding properties of carbon dots.

Related technologies are described in Nanoscale Research Letters, 2014, 9(1), 397, KR 2016 0003488 A, Acta Biomaterialia, 2015, 26, 295-305, US 2013/156856A1, Scientific Reports, 2015, 5(1), Jang, M et al.: "Is the Chain of Oxidation and Reduction Process Reversible in Luminescent Graphene Quantum Dots?", Small, vol. 11, no. 31, 2015, pages 3773-3781, Wu, M et al.: "Preparation of functionalized water-soluble photoluminescent carbon quantum dots from petroleum coke", Carbon, vol. 78, 2014, pages 480-489, WO 2014/179708A1, Sun, H et al.: "Improvement of Photoluminescence of Graphene Quantum Dots with a Biocompatible Photochemical Reduction Pathway and Its Bioimaging Application",

APPL. MATER. INTERFACES, vol. 5, 2013, pages 1174-1179, LV QING ET AL: "Characteristics of sequential targeting of brain glioma for transferrin-modified cisplatin liposome", INTERNATIONAL JOURNAL OF PHARMACEUTICS, vol. 444, no. 1, 21 January 2013, pages 1-9, Shadabul Haque ET AL: "Nanostructure-based drug delivery systems for brain targeting", Journal Drug Development and Industrial Pharmacy, vol. 38, no. 4, 28 September 2011, pages 387-411, Li Jingyan ET AL: "PLA/PLGA nanoparticles for delivery of drugs across the blood-brain barrier", Nanotechnology reviews, vol. 2, no. 3, 5 March 2013, pages 241-257.

Accordingly, it would be advantageous to provide non-toxic carbon dots useful for biological applications, that demonstrate one or more advantages such as inhibiting changes in protein and peptide conformations, and the ability to permeate the blood-brain barrier.

### Summary of the Invention

One aspect of the invention provides a method of forming a blood-brain barrier permeating solution, according to claim 1.

For the compositions and methods described herein, optional features, including but not limited components, compositional ranges thereof, conditions, and steps are contemplated to be selected from the various aspects, embodiments, and examples provided herein.

Further aspects and advantages will be apparent to those of ordinary skill in the art from a review of the following detailed description, taken in conjunction with the drawings and examples.

### Brief Description of the Drawings

For further facilitating the understanding of the present invention one drawing figure is appended hereto.

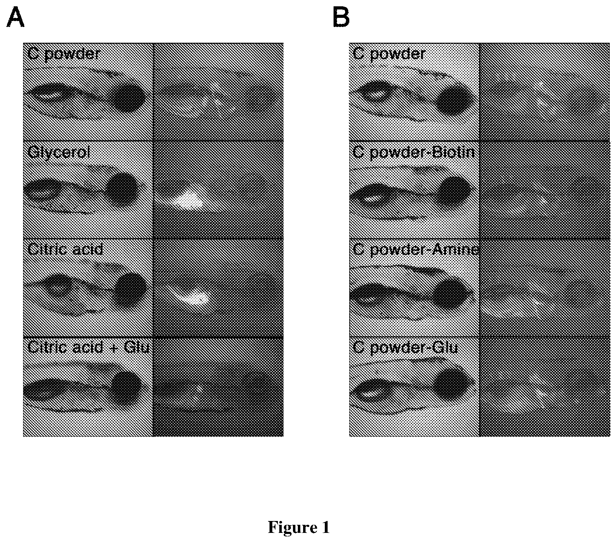

Figure 1 is an image demonstrating that carbon dots prepared according to the disclosure have a binding affinity for calcified bone in live animals while carbon dots prepared from other starting materials do not have a binding affinity for calcified bone in live animals.

### Detailed Description

One aspect of the disclosure provides a method of forming carbon dots, the method including admixing carbon nanopowder with sulfuric acid and nitric acid to form a carbon nanopowder mixture, heating the carbon nanopowder mixture with reflux to form a refluxed carbon nanopowder mixture and then cooling the refluxed carbon nanopowder mixture, neutralizing the refluxed carbon nanppowder mixture to form a neutralized carbon nanopowder powder mixture comprising solubilized carbon dots, isolating the solubilized carbon dots from the neutralized carbon nanopowder mixture to form a carbon dots solution, dialyzing the carbon dots solution, and separating a solvent of the solution from the carbon dot solution to obtain the solid carbon dots.

As used herein and unless specified otherwise, "carbon powder" refers to carbon powders having a particle size greater than 100 nm and carbon nanopowders having a particles size of 100 nm or less.

In some embodiments, the carbon dots have an average diameter of less than about 10 nm, optionally about 6 nm or less. The sulfuric acid and nitric acid are provided in a ratio greater than about 1:1 (v/v), optionally about 1.1:1 to about 5:1 (v/v).

In embodiments, neutralizing the refluxed carbon nanopowder mixture comprises adding a base to the mixture to form the neutralized carbon nanopowder mixture comprising solubilized carbon dots and at least one of a sulfate salt and/or a nitrate salt. Optionally, the base is an alkali hydroxide, for example, selected from the group consisting of sodium hydroxide, potassium hydroxide, lithium hydroxide, and combinations of the foregoing. In embodiments, the base may be an over-saturated solution of an alkali hydroxide. In embodiments, the base may be an over-saturated solution of sodium hydroxide.

In embodiments, isolating the soluble carbon dots from the neutralized carbon nanopowder mixture comprises crystallizing the at least one of the sulfate salt and/or nitrate salt and removing the salt from the neutralized carbon nanopowder mixture. In embodiments, crystallization of the salt includes adding a sodium sulfate crystal to the neutralized carbon nanopowder mixture to initiate crystallization of the salt. In embodiments, crystallization of the salt may include reducing the volume of the solvent of the neutralized carbon nanopowder mixture. Optionally, the salt may be removed by filtration.

Isolating the soluble carbon dots from the neutralized carbon nanopowder mixture comprises removing impurities from the neutralized carbon nanopowder mixture. Optionally, the impurities may be removed by extraction with an organic solvent. In embodiments, the neutralized carbon nanopowder mixture may be filtered to remove unreacted carbon nanopowder. Optionally, the neutralized carbon nanopowder mixture may be filtered to remove unreacted carbon nanopowder prior to crystallizing the at least one sulfate salt and/or nitrate salt.

In embodiments, the carbon dots solution is centrifuged prior to dialyzing. In embodiments, the carbon dots solution may be dialyzed with about 4 L of deionized water for about five days. In embodiments, the solvent is separated from the carbon dot solution to obtain the solid carbon dots by evaporation, for example, concentrating the carbon dot solution and evaporating residual solvent.

Another aspect of the disclosure provides a method of forming a blood-brain barrier permeating solution, the method including covalently conjugating carbon dots formed according to methods of the disclosure to an organic compound target to form carbon dot-organic compound target conjugates and admixing the conjugates with a solvent to form the blood-brain barrier permeating solution.

In embodiments, the organic compound target is selected from the group consisting of transferrin, dye-labeled transferrin, and combinations thereof.

In embodiments, the carbon dot is not a surface-modified carbon dot. In embodiments, the carbon dot comprises a surface-modified carbon dot. Optionally, the surface modification may be selected from the group consisting of neutral biotin, positively charged amine groups, or negatively charged carboxyl groups.

As used herein and unless specified otherwise a carbon dot that is "not a surface-modified carbon dot" is a carbon dot prepared according to the disclosure which, after isolation of the solid carbon dot powder, is not intentionally further modified at the carbon dot surface.

"Comprising" as used herein means that various components, ingredients or steps that can be conjointly employed in practicing the present disclosure. Accordingly, the term "comprising" encompasses the more restrictive terms "consisting essentially of" and "consisting of." The present compositions can comprise, consist essentially of, or consist of any of the required and optional elements disclosed herein.

All ranges set forth herein include all possible subsets of ranges and any combinations of such subset ranges. By default, ranges are inclusive of the stated endpoints, unless stated otherwise. Where a range of values is provided, it is understood that each intervening value between the upper and lower limit of that range and any other stated or intervening value in that stated range, is encompassed within the disclosure. The upper and lower limits of these smaller ranges may independently be included in the smaller ranges, and are also encompassed within the disclosure, subject to any specifically excluded limit in the stated range. Where the stated range includes one or both of the limits, ranges excluding either or both of those included limits are also contemplated to be part of the disclosure.

### Method of Preparing Carbon Dots

The present application includes techniques to form of carbon dots (also termed "C-Dots" herein) using a "top-down" approach. More specifically, fluorescent C-Dots were successfully prepared using carbon powder in some examples and carbon nanopowder in other examples. The size of the carbon powder is not particularly limited, and may be, for example, 1000 nm or less. The carbon powder is typically not water soluble.

In general, the method of preparing carbon dots of the disclosure include the steps:
(a) admixing carbon powder with acid to form a carbon powder mixture;
(b) heating the carbon powder mixture;
(c) cooling the carbon powder mixture;
(d) neutralizing the carbon powder mixture comprising the formed carbon dots;
(e) isolating the solubilized carbon dots from the salts formed in the neutralization reaction, any impurities present in the solution, and/or any residual starting materials;
(f) dialyzing the carbon dot solution;
(g) separating the solvent from the carbon dot solution to form solid carbon dots.

The steps (a) to (g) of preparing the carbon dots are described in detail below.

To form the carbon dots of the disclosure, the carbon powder is oxidized using acid. The carbon powder is first admixed with acid to form a carbon powder mixture. Suitable acids for the preparation of carbon dots include strong oxidizing acids including, but not limited to mixtures of sulfuric acid and nitric acid, and chromic acid. Sulfuric acid and nitric acid may be provided in a ratio of greater than about 1:1 by volume (v/v), or greater than about 2:1, or greater than about 3:1 and up to about 10:1, or up to about 5:1, or up to about 4:1, for example, about 1.1:1, about 1.2:1, about 1.5:1, about 2:1, about 2.5:1, about 3:1, about 3:5:1 or about 4:1 (v/v). Without intending to be bound by theory, it is believed that a sulfuric acid and nitric acid mixture having a 1:1 (v/v) ratio or less is not strong enough to oxidize the carbon powder to generate enough carboxyl groups at the surface to provide water soluble carbon dots.

For example, in the mixture of sulfuric acid and nitric acid (3:1, v/v), carbon nanopowder was oxidized to quantum size with diameters between 1.5 and 6 nm. The acid ratio was important, as a 1:1 mixture or nitric acid alone could not synthesize C-Dots under the same conditions. The as-prepared C-Dots were already water-soluble and fluorescent. Similar C-Dots were also prepared from carbon powder using the same procedures.

The amount of acid may be any amount suitable to wet the carbon powder to provide a carbon powder suspension (mixture). For example, the acid may be provided in an amount selected such that the carbon powder mixture has a carbon powder concentration of about 20 mg/mL, for example, in a range of about 10 mg/mL to about 50 mg/mL, or about 10 mg/mL to about 40 mg/mL, or about 10 mg/mL to about 30 mg/mL, or about 20 mg/mL to about 40 mg/mL.

The carbon powder mixture is then heated with reflux to form a refluxed carbon nanopowder mixture. The carbon powder mixture may be heated to, for example, a temperature greater than about 100°C, or greater than about 110°C and up to about 120°C or up to about 115°C. The heating may be maintained for any duration of time suitable to oxidize the carbon powder. The carbon powder mixture may be heated with reflux for at least 1 h, at least 3 h, at least 6 h, at least 9 h, at least 12 h, or at least 15 h, and up to about 72 h, up to about 60 h, up to about 48 h, up to about 36 h, up to about 24 h, up to about 20 h, or up to about 16 h.

After heating, the refluxed carbon powder mixture is cooled prior to neutralization. The cooling of the carbon powder mixture is not particularly limited. The carbon powder mixture may be cooled to ambient temperature and the reaction flask may then be placed in an ice bath prior to neutralization. The neutralization step is generally highly exothermic and, therefore, cooling of the carbon powder mixture after reflux is advantageous to control the exotherm of the subsequent neutralization.

The refluxed carbon powder mixture is neutralized to form a neutralized carbon powder mixture comprising solubilized carbon dots. The neutralized carbon powder mixture further comprises salts of sulfuric acid and/or nitric acid. The base used to neutralize the sulfuric acid and nitric acid is not particularly limited. Suitable bases may include alkali hydroxides, for example, sodium hydroxide, potassium hydroxide, lithium hydroxide, and combinations of the foregoing. The base may be added as a dilute solution, a saturated solution, or an over-saturated solution. An over-saturated solution may be advantageous because less solvent will need to be removed from the neutralized solution to obtain solid carbon dots.

The solubilized carbon dots are isolated from the salts formed in the neutralization reaction, any impurities present in the solution, and/or any residual starting materials. The salts formed during neutralization may be removed according to any method known in the art. For example, the salts may be crystallized out of the neutralized carbon powder mixture and the mixture filtered to separate the solid salt and the liquid supernatant. To facilitate crystallization, the neutralized carbon powder mixture may be concentrated by reducing the volume of the solvent, the concentrate then cooled to initiate precipitation of the salt from the mixture. The mixture may be concentrated by heating to evaporate at least a portion of the solvent at any suitable temperature, for example, in a range of about 70°C to about 110°C, or about 70°C to about 100°C, or about 75°C to about 95°C, or about 75°C to about 90°C, or about 75°C to about 85°C. Additionally or alternatively, the mixture may be concentrated by including an appropriate seed crystal to the neutralized carbon powder mixture. For example, a sodium sulfate crystal may be added to initiate crystallization of the neutralization salts.

Impurities may be removed from the neutralized carbon powder mixture by any suitable means known in the art. Common methods of removing impurities include, but are not limited to, centrifugation and extraction. Liquid/liquid extraction may be performed (e.g., with a separatory funnel) by mixing the aqueous neutralized carbon powder mixture with an organic solvent. One of ordinary skill in the art will readily appreciate that a suitable organic solvent will be one that is immiscible with the aqueous phase. Suitable organic solvents include, but are not limited to chloroform, dichloromethane, carbon tetrachloride, and ethyl acetate. Centrifugation may be performed at any stage after neutralization. In embodiments, centrifugation is performed after liquid/liquid extraction but prior to dialyzing of the isolated carbon dot solution.

Residual starting materials may also be removed from the neutralized carbon powder during isolation of the solubilized carbon dots. Residual starting materials may be removed by any method known in the art. Specifically, because the starting carbon powder is not water soluble, but the formed carbon dots are water soluble, residual, unreacted carbon powder may be removed by filtration. Residual carbon powder may be removed at any stage after the neutralization of the carbon powder mixture, for example, before or after crystallizing out the neutralization salts or before or after extracting out impurities.

The carbon dot solution including the isolated, solubilized carbon dots may be dialyzed prior to separating the solvent from the carbon dots to remove any residual sulfate and/or nitrate salts, unreacted acid, unreacted carbon powder, and any impurities formed as a byproduct of the reaction. The duration and volume of deionized water used to dialyze the carbon dot solution are not particularly limiting. For example, the carbon dot solution may be dialyzed for at least 1 day, at least 3 days, or at least 5 days and up to 10 days, up to 8 days, or up to 6 days. The deionized water may be changed after intervals of about 2 h, about 4 h, about 6 h, about 8 h, or about 10 h. The volume of water provided for each dialysis interval may be at least about 3 L, at least about 4 L, at least about 5 L or less than about 8 L, less than about 7 L, or less than about 6 L. Without intending to be bound by theory, it is believed that the higher the frequency of water changes and the longer the overall dialysis period, the more pure the resulting carbon dots will be.

Solid carbon dots may be obtained by removing the solvent from the carbon dot solution. The solvent may be removed according to any methods known in the art. For example, the carbon dot solution may be concentrated and then the residual solvent evaporated off. Concentrating the carbon dot solution may be performed by heating the carbon dot solution to a temperature in a range of about 70°C to about 110°C, or about 70°C to about 100°C, or about 75°C to about 95°C, or about 75°C to about 90°C, or about 75°C to about 85°C. Residual solvent may be evaporated off at reduced pressure, for example, using a rotoevaporator (rotovap).

The solid carbon dots may have any size suitable for the intended application. For example, a solid carbon dot intended for use in a blood-brain barrier permeable membrane must be small enough to pass through the BBB by receptor-mediated endocytosis, as described below. Suitably the carbon dots have an average particle diameter below 10 nm, for example 8 nm or less, 6 nm or less, 4 nm or less, or 2 nm or less, for example, from about 1 to about 8 nm, from about 2 to about 6 nm, or about 4 nm. The as prepared carbon dots have carbon cores with rich surface carboxylic groups on the surface as well as rich sp² carbons, and may carrier negative charges on the carboxyl groups. Thus, the carbon dots may be modified at the surface and/or conjugated with organic compounds and/or loaded with drugs through conjugation at the carboxylic groups with compounds having active functional groups (non-limiting examples of active functional groups include amine, alcohol, carboxyl, and thiol), or noncovalent interactions such as adsorption, electrostatic interaction, or pi-pi interactions.

In an example, aliquots containing sulfuric acid (9 mL) and nitric acid (3 mL) were added to 250 mg of carbon nanopowder in a flask. The mixture was heated with reflux to about 110 °C for 15 h in a sand bath. After cooling, over-saturated sodium hydroxide solution was added to neutralize the solution in an ice bath. The mixture was filtered to remove the unreacted carbon powder. An ice bath was then used to crystallize the salt formed. The solution could be supersaturated and a piece of sodium sulfate crystal may be necessary to start crystallization. The contents were filtered again to obtain a dark-brown supernatant solution. This procedure was repeated again to further remove the supersaturated salt. The supernatant solution was transferred to a beaker, and its volume reduced to about 25 mL by evaporating at 75-85 °C to concentrate it. The solution was cooled in an ice bath to remove the salt crystals and obtain the dark brown supernatant solution. Chloroform (15 mL) was added to extract impurities into the organic phase. We reserved the aqueous phase and repeated the extraction procedure twice. Then, the solution was centrifuged at 3000 rpm for 30 min to remove any precipitates. We transferred the solution to a molecular weight cutoff (MWCO) 3500 dialysis bag and dialyzed it with 4 L of deionized water for 5 days; the deionized water was changed every 4-10 h. Then, the solution was concentrated by heating it to 75-85 °C, until about 25 mL remained. Finally, the water was evaporated using a rotovap to yield 27.4 mg of black powder as C- Dots.

### Method of Inhibiting Protein/Peptide Fibrillation (not according to the invention)

The present application further provides for inhibiting biological operation through the use of carbon dots, e.g., inhibiting peptide or protein fibrillation by affecting them with carbon dots. Peptide or protein fibrillation in the extracellular space of tissues plays a significant role in the development of several serious human diseases, such as Alzheimer's disease, type 2 diabetes and Parkinson's disease. These peptide or protein fibrils feature well-defined cross-β-sheet structures through misfolding of the native conformations. Fibrillation typically follows a nucleation-growth pattern, including initial formation of small nuclei through oligomerization, and then elongation of the fibrils via protofibril formation. The intermediate oligomeric species and the mature fibrils have cytotoxicity, provoking the death of related cells. Thus, prevention and therapeutic strategy for the diseases associated with peptide or protein fibrillation is to inhibit or delay the fibrillation process.

Insulin fibrils are found in some patients with type 2 diabetes after insulin infusion and repeated injection. Insulin is one of the therapeutic proteins with the largest production volume but its fibrillation is still a challenging problem in production, storage, and delivery of the protein.

Protein and peptide fibrillation may be inhibited by admixing the protein or peptide with carbon dots of the disclosure. In embodiments, admixing the carbon dots with a protein or peptide comprises administration of a concentrated solution of the carbon dots to a patient in need of inhibition of a protein or peptide. In embodiments, the admixing the carbon dots with a protein or a peptide is done prior to administration of the carbon dots to a subject. Optionally, the protein or peptide and the carbon dots are admixed in solution to form a concentrated solution. The concentrated solution may be administered to a subject to treat the subject.

Concentrated solutions may include a concentration of carbon dots of at least 2 µg/mL, at least 4 µg/mL, at least 6 µg/mL, at least 8 µg/mL, or at least 10 µg/mL and up to about 20 µg/mL, up to about 18 µg/mL, up to about 16 µg/mL, up to about 14 µg/mL, or up to about 12 µg/mL. Without intending to be bound by theory, it is believed that the carbon dots of the disclosure inhibit protein and peptide fibrillation in a concentration-dependent manner. Accordingly, the carbon dots may be provided in an amount sufficient to inhibit the fibrillation of a protein or peptide for at least 5 h, at least 8 h, at least 12 h, at least 1 day, at least 3 days, or at least 5 days and up to about 30 days, up to about 25 days, up to about 20 days, up to about 15 days, up to about 10 days, or up to about 8 days while being incubated at a temperature of 65°C. Without intending to be bound by theory, it is believed that because incubation at a temperature of 65°C is adverse to a protein or peptide, the duration of inhibition of fibrillation would be expected to increase for a protein or peptide admixed with an equivalent amount of carbon dots but stored under less harsh conditions, e.g., ambient conditions.

For example, the effects of C-Dots on peptide or protein fibrillation are examined. In an example, human insulin was selected as a model to investigate the effect of C-Dots on insulin fibrillation. Water-soluble fluorescent C-Dots with sizes less than 6 nm were prepared from carbon powder and characterized by UV-vis spectroscopy, fluorescence, Fourier transform infrared spectrophotometry, X-ray photoelectron spectrometry, transmission electron micros-copy, and atomic force microscopy. These C-Dots were able to efficiently inhibit insulin fibrillation in a concentration-dependent manner. The inhibiting effect of C-Dots was even observed at 0.2 µg/mL. Importantly, 40 µg/mL of C-Dots prevent 0.2 mg/mL of human insulin from fibrillation for 5 days under 65 °C, whereas insulin denatures in 3 h under the same conditions without C-Dots. Cytotoxicity study shows that these C-Dots have very low cytotoxicity. Therefore, these C-Dots are able to inhibit insulin fibrillation in biological systems and may be used in the pharmaceutical industry for the processing and formulation of insulin. More details on the formation of carbon dots and their use in peptide or protein fibrillation inhibition are provided in Attachment A and can be found in the Examples, below.

As demonstrated in the examples, the carbon dots of the disclosure have a greater inhibiting effect on human insulin fibrillation when added at the earlier stage of nucleation. Without intending to be bound by theory, it is believed that the inhibiting effect is likely due to the interaction between the carbon dots and the insulin species (monomers and oligomers) before the critical nucleation concentration is reached. Once reached, the carbon dots do not change the kinetics of fibrillation. Further, without intending to be bound by theory, it is believed that the interaction of the carbon dots with the human insulin species is attributed to weak interactions such as hydrogen bonding, hydrophobic interaction, and van der Waals interactions due to the complicated surface nature of carbon dots. These interactions may be strong enough to adsorb insulin species onto the surfaces of carbon dots, slowing down the self-aggregation and nucleation of human insulin at the early stage of fibrillation. Electrostatic interaction is not expected to contribute much. Due to the protonation of the carboxylic group at pH 1.6, carbon dots do not have much negative charge on their surfaces.

It is well accepted that peptides and proteins may share a common molecular mechanism to develop fibrils, regardless of their sources, sequences, and functions. Further because other proteins and peptides are formed of amino acids having the same functional groups present on insulin, the carbon dots would be expected to interact with other proteins and peptides through weak interactions such as hydrogen bonding, hydrophobic interaction, and van der Waals interaction in the same way as insulin. Accordingly, the inhibiting of insulin fibrillation by the carbon dots of the disclosure is expected to similarly inhibit the fibrillation of other proteins and peptides.

### Blood-Brain Barrier Permeating Composition

The disclosure further provides a method of transporting carbon dots of the disclosure through the blood-brain barrier in a blood-brain permeating solution. The central nervous system (CNS), consisting of the brain and spinal cord, is responsible for integrating sensory information and responding accordingly. The CNS is protected by the complex and highly regulated blood-brain barrier (BBB) which serves as a physiological checkpoint to allow the entry of selected molecules from the blood circulation into the CNS. The BBB is primarily composed of capillary endothelial cells, which are closely interconnected by tight intercellular junctions. Thus, the BBB is an obstacle for the delivery of therapeutic molecules from the blood to the CNS. Studies show that more than 98% of small-molecule drugs and practically 100% of large-molecule drugs targeted for CNS diseases do not readily cross the BBB and, therefore, current treatments for CNS diseases remain extremely limited.

The blood-brain barrier permeating solution generally comprises a carbon dot of the disclosure covalently conjugated to an organic compound target. The carbon dot-organic compound target conjugate can permeate the blood-brain barrier via receptor-mediated endocytosis. Thus, in embodiments the organic compound target may be a ligand that is specific to a receptor found at the blood-brain barrier. Ligands specific to receptors at the blood-brain barrier include, but are not limited to, ligands specific to transferrin receptors, insulin receptors, mannose 6-phosphate receptors (insulin-like growth factor II), low density lipoprotein receptor-related protein 1 receptors, low density lipoprotein receptor-related protein 2 receptors, leptin receptors, thiamine receptors, glutathione receptors, opioid receptors, p75 neurotrophin receptor, GT1b polysialogangliosides, GPI anchored protein receptor, and diphtheria toxin receptor (heparin binding epidermal growth factor-like growth factor). Organic compound targets may be selected from the group consisting of transferrin, insulin, human melanoma antigen p97, low density lipoproteins, receptor-associated protein, polysorbate 80-coated nanoparticles, angiopeps (e.g., angiopep 2), leptin, thiamine, glutathione, synthetic opioid peptide, rabies virus glycoprotein, tetanus toxin, ten-eleven translocation methylcytosine dioxygenase 1 (TET 1), G23 peptide, TAT peptide and nontoxic mutants of diphtheria toxin (CRM197). The organic compound target may also be labeled to enhance imaging of the carbon-dot-organic compound conjugate within a subject. Any compounds used in medicine as a contrast media for imaging applications are suitable imaging labels. Non-limiting examples of an imaging label may be a fluorescent dye, e.g., fluorescein, CF^{™} dyes series, and Alexa Fluor^{®} dyes series, or a radiocontrast reagent, e.g., iodine, barium, gandolinium.

Methods of conjugating organic compound targets to carbon dots are well known in the art. Any method of covalently attaching the organic compound target to the carbon dot is suitable. For example, classical carbodiimide chemistry (e.g., EDC/NHS, using 1-ethyl-3-(3-dimethylaminopropyl)carbodiimide) (EDC) and *N-*hydroxysuccinimide (NHS) or sulfo-(NHS) may be used to conjugate amino- or alcohol-containing organic compounds to carbon dots.

In another example, the present techniques demonstrate the formation and use of carbon dots ("C-Dots") in crossing the blood-brain barrier, specifically with transferrin conjugated carbon dots. The applicants have identified that to utilize the inhibiting effect of C-Dots on protein (or peptide) fibrillation, C-Dots should be delivered first into the CNS by crossing the BBB. While C-Dots have shown potential for drug delivery and treatment for some CNS related diseases, for effective drug delivery and treatment, it is desirable to deliver C-Dots to the CNS by crossing the blood-brain barrier (BBB), which blocks therapeutic agents from reaching the pathological tissues in the CNS. Before the present work, conventional techniques failed to demonstrate C-Dots or C-Dots conjugates crossing the BBB to enter the CNS. With the present techniques, C-Dots were covalently conjugated to transferrin and dye labeled transferrin and demonstrated as crossing the BBB, via the transferrin receptor-mediated delivery. The experiments were performed using a zebrafish model and suggested that the transferrin conjugated C-Dots could enter the CNS by crossing the BBB, while C-Dots alone could not. The BBB in zebrafish, mice and humans is very similar and, therefore, the findings in zebrafish are expected to be applicable to mice and humans.

### Bone Binding Compositions (not according to the invention)

Materials used for *in vivo* bone imaging using fluorescence microscopy are extremely rare. *In vivo* imaging by fluorescence microscopy require the fluorophore to have bone specificity with no or limited non-specific binding to other cells or organs, the probe must be able to distinguish between mostly cartilaginous and mostly calcified bone, the emission of the fluorophores at the target sites must be strong enough to yield the signals, the fluorophores need to be biocompatible with no or minimal cytotoxicity, and the target fluorophores need to be optimized simultaneously for shorter delivery time after administration but longer staining time.

The carbon dots of the disclosure have advantageously been found to bind to calcified bones in live animals with high affinity and specificity. Binding resulted in a strong enhancement of luminescence that was not observed in other tissues, including non-calcified endochondral elements. Thus, the carbon dots of the disclosure may be used for diagnostic and/or therapeutic purposes.

Therapeutic uses of the carbon dots are not particularly limited. In general, the carbon dot of the disclosure can be used in a method of delivering a drug to a bone, the method including loading a carbon dot of the disclosure with a drug to form a carbon dot loaded with the drug and administering the carbon dot loaded with the drug to a subject.

The drug that is loaded onto the nanoparticle is not particularly limited. Any drug that can conjugate to the carbon dot may be delivered to a calcified bone. The drug may be a drug for treating bone mineralization disease (such as, for example, osteoporosis or heterotopic ossification). The drug may be a drug for treating energy metabolic diseases of bone origin. Because the skeleton, together with the brain, pancreas, gut and liver is part of the endocrine circuit that regulates energy metabolism (and thus growth and obesity), the carbon dots of the disclosure may be used to deliver drugs to treat energy metabolism defects and diseases by specifically targeting the bone component of the endocrine system. The drug may also be a drug to treat bone and blood cancers, as well as tumors that metastasize to bones. Bone cancer can develop in any type of bone tissue (e.g., osteosarcoma and Ewing Sarcoma osteoid tissue, chondrosarcoma in cartilaginous tissue), blood cancer can develop in bone marrow (e.g., multiple myeloma), and bone is a common site of metastasize cancer (e.g., metastatic breast cancer). Carbon dots prepared according to the disclosure may be used to deliver chemotherapy agents and other drugs to bones to treat cancer and other diseases. The drug may also be an antibiotic such as, but not limited to, penicillin and derivatives thereof, and ciprofloxacin and derivatives thereof. Bones can be infected with bacteria (e.g., *Staphylococcus aureus*) in individuals whose immune system has been weakened by disease or illness (e.g., diabetes, arthritis, AIDS, etc.) or whose bones have been exposed to the environment (e.g., open fractures, or replacement surgery such as hip, knee, etc.). Carbon dots may be used to specifically treat infections in bones.

Without intending to be bound by theory, it is believed that the rich surface of carboxylic groups provides for the high affinity and specificity towards bones. Further, without intending to be bound by theory, it is believed that even after conjugation of the carbon dots with drugs, a sufficient amount of carboxylic groups remain which allows the modified carbon dot to bind to bone. Carbon dots of the disclosure not modified at the surface, as well as carbon dots of the disclosure that have been conjugated with amine, glutamic acid, and biotin, all bind to bone in live animals and specifically to bone and not to other tissues, such as non-calcified bone matrix (extracellular matrix). Other carbon dot surface modifications are possible (e.g., thiol) and similar binding of modified carbon dots are expected to occur as shown with amine, glutamic acid, and biotin.

A drug may be functionally attached to the carbon dots because of the tunable surface functionalities of the carbon dots, and any active functional group present on the drug. As demonstrated with the carboxyl and amine groups, the surface of the carbon dots may be modified without loss of affinity or specificity. Further, if a drug has a functional group that is chemically incompatible with the functional groups at the surface of the unmodified carbon dots, the carbon dot may be modified with a different surface group that is compatible with the functionality of the drug. Additionally, a drug may be loaded onto carbon dots of the disclosure through noncovalent interactions.

Methods of conjugating drugs to carbon dots are well known in the art. For example, classical EDC/NHS coupling chemistry may be used.

Diagnostic uses of the carbon dots of the disclosure are not particularly limited. The carbon dots of the disclosure may be used as an imaging reagent of fractures and microfractures. The intrinsic fluorescent properties of the carbon dots allow visualization of calcified bones. The carbon dots may also be used as a delivery vehicle of imaging contrast reagents to bone. The unique bone affinity of the carbon dots allows delivery of contrast reagents that can be used to visualize bone structure for any known detection method (e.g., X-Rays, computer tomography, MRI, etc.).

The carbon dot may not have a surface-modification. In embodiments, the carbon dot comprises a surface-modified carbon dot. Suitable surface modifications may be selected from the group consisting of neutral biotin, positively charged amine groups, or negatively charged carboxyl groups. A surface modification may be used to promote loading of the drug on the carbon dot.

Surprisingly, it was found that bone binding in live animals is not a general property of carbon dots. Using a zebrafish model,, it was advantageously found that carbon dots prepared according to the method of the disclosure were able to bind to calcified bone (Figure 1A (C powder)), as were carbon dots prepared according to the method of the disclosure that were further surface modified with neutral biotin, positively charged amino groups and negatively charged carboxyl groups (Figure 1B). Carbon dots prepared according to the method of the disclosure and surface modified were found to have the same affinity of unmodified carbon dots for calcified bone. Further, the carbon dots and modified carbon dots of the disclosure bind specifically to calcified bone and not to other tissues, such as non-calcified bone matrix (extracellular matrix). In contrast, carbon dots prepared according to known methods, i.e. prepared from glycerol or citric acid, were not able to bind to the calcified bone as shown in Figure 1A (Glycerol and Citric Acid), even if the surface was modified with glutamic acid to increase the amount of calcium-binding carboxyl groups (Figure 1A Citric acid + Glu). Rather, the fluorescence of the carbon dots prepared according to known methods is observed in the gut and detoxifying organs (liver, pronephros (rudimentary kidneys)). Without intending to be bound by theory, it is believed that the specificity and affinity of the carbon dots of the disclosure to calcified bone is attributed to the combination of the purity of the carbon dots and the rich surface of carboxyl and alcohol groups. The carbon dots of the disclosure have a pure carbon core with rich carboxyl and alcohol groups at the surface (generally negatively charged). In contrast, carbon dots prepared according to known methods have a less pure carbon core (formed by polymerization and carbonization) and other, different, functional groups at the surface, depending on the method of preparation. For example, carbon dots formed with glycerol or citric acid include negatively charged carboxyl groups as well as positively charged amine groups.

Carbon dots may be administered to a bone by any suitable method known in the art. Non-limiting examples of administration include injection into the blood, intraperitoneal injection, and local delivery by direct exposure of a wound to carbon dots. When injected into the blood stream, the carbon dots circulate in the organism and then attach to bones. Circulating carbon dots are cleared from the blood as they attach to bones. When directly injected into the body cavity carbon dots are distributed to bones from the peritoneum via the circulatory system. Intraperitoneal injection would be suitable for delivering chemotherapy treatments in humans. When carbon dots are delivered locally, the carbon dots bind to directly exposed, wounded bones.

Retention of carbon dots of the disclosure by zebrafish was very stable, long lasting, with no detectable toxicity and was independent of the administration method.

The carbon dots and methods in accordance with the disclosure can be better understood in light of the following examples.

### Examples

### Example 1: Preparation of Carbon Dots

Carbon dots of the disclosure were prepared as followed. Sulfuric acid (9 mL) and nitric acid (3 mL) were added in aliquots to 250 mg of carbon nanopowder or carbon powder in a flask to form a carbon powder mixture. The mixture was refluxed at about 110 °C for 15 h in a sand bath. The refluxed carbon powder mixture was then cooled. While the flask was in an ice bath, over- saturated sodium hydroxide solution was added to neutralize the cooled, refluxed carbon powder mixture. The mixture was filtered to remove unreacted carbon powder. The salt formed by the neutralization reaction was then removed as follows. A sodium sulfate seed crystal was added to the mixture and the mixture was cooled in an ice bath to promote precipitation/crystallization of the salt. The contents were filtered to remove the solid salt that formed and a dark-brown supernatant solution including solubilized carbon dots was obtained. The precipitation/crystallization/filtration was repeated as necessary to remove all of the salt. The supernatant solution including the solubilized carbon dots was transferred to a beaker, and its volume reduced to about 25 mL by evaporating at 75- 85 °C at atmospheric pressure. A liquid/liquid extraction was used to extract impurities from the solubilized carbon dot solution. In particular, chloroform (15 mL) was added to extract impurities into the organic phase. The aqueous phase was collected and the extraction procedure repeated. The solution was then centrifuged at 3000 rpm for 30 min to remove any precipitates. The solution was transferred to a molecular weight cutoff (MWCO) 3500 dialysis bag and dialyzed with 4 L of deionized water for 5 days; the deionized water was changed every 4-10 h. The resulting solution was concentrated by heating it to 75-85 °C, until about 25 mL remained. Finally, the remaining water was evaporated using a rotovap (or equivalent) to yield 27.4 mg of solid black powder as water-soluble carbon dots.

The prepared carbon dots were characterized by ultraviolet-visible spectroscopy (US-Vis) in a 1 cm cell using a Shimadzu UV-2600 spectrometer, or equivalent. The Fourier transform infrared (FTIR) spectrum was recorded on a Perkin Elmer Frontier, or equivalent, using the solid powder of carbon dots. Fluorescent emission spectra of the carbon dots were measured in aqueous solution by a Horiba Jobin Yvon Fluorolog-3, or equivalent, with a slit width of 5 nm for both excitation and emission. X-ray photoelectron spectroscopy (XPS) was performed using a Perkin-Elmer PHI 560 ESCA system, or equivalent, with a double-pass cylindrical mirror analyzer operated at 225 W and 12.5 KV using a Mg Kα anode and a photon energy of hυ = 1253.6 eV. Core levels of the carbon 1s orbitals and oxygen 1s orbitals were scanned and intensities were normalized according to their respective atomic sensitivity factors. Microscopic images of carbon dots were obtained on an Agilent 5420 atomic force microscope, or equivalent, using a tapping mode and a JEOL 1200X TEM.

Broad strong UV-Vis absorption peaks were found in the range of 200-400 nm. The carbon dots demonstrated excitation wavelength dependent emission. The maximal emission of the carbon dots was around 580 nm when excited at 540 nm. The emission peak shifted to about 500 nm when excited at 360 nm. FTIR analysis showed peaks at about 3380 (OH), 1715 (C=O), 1582 (C=C), 1236 (C-O-C) and 1085 cm⁻¹ (C-O). These bonds were further confirmed by XPS, which reveals 54.6% carbon, 43.8% oxygen, and other trace elements on the carbon dot surface. An XPS peak at 289.7 eV was attributed to carboxylic acid groups, which comprised 23% of the oxygen signal. A 285.9 eV peak was attributed to the C-C/C=C bonds, a 532.7 eV peak was attributed to hydroxyl oxygen from water, and a 534.0 eV peak was attributed to carbonyl oxygen. The TEM images showed spherical carbon dots having diameters distributed between 1.5 and 6 nm, with an average of 4 nm.

Thus, Example 1 shows preparation and characterization of carbon dots according to the disclosure.

### Comparative Example 2: Attempted Preparation of Carbon Dots (reference)

Carbon dots were prepared as described in Example 1, except in one preparation a 1:1 (v/v) mixture of sulfuric acid and nitric acid was used in place of the 3:1 (v/v) mixture and in a second preparation, only nitric acid was used. Carbon dots did not form in either the preparation using a 1:1 mixture of sulfuric acid and nitric acid or the preparation using nitric acid alone. Thus, Comparative Example 2 demonstrates that carbon dots of the disclosure are not obtained when the ratio of sulfuric acid to nitric acid is 1:1 (v/v) or less.

### Example 3: Effect of Carbon Dots on Insulin Fibrillation (reference)

1 mg/mL of human insulin (about 5.8 kDa molecular weight) stock was prepared in hydrochloric acid aqueous solution (pH 1.6) with 0.1 M sodium chloride (NaCl). The solution was filtered through a 0.2 µm pore size filter. Carbon dots prepared according to Example 1 were dissolved in water at a concentration of 1 mg/mL. The insulin stock solution was mixed with the carbon dot solution to prepare several samples having insulin concentration of 0.2 mg/mL with one of 0, 0.2, 2, or 10 µg/mL of carbon dots using 0.1 M NaCl solution at pH 1.6. The samples were incubated at 65°C. Aliquots of samples were taken every 30 min, diluted with Thioflavin T (ThT, 40 µM at pH 1.6, 0.1 M NaCl) to 0.1 mg/mL of protein and 20 µM of ThT. The ThT fluorescence was recorded on a Fluorolog-3 spectrofluorometer, or equivalent, at excitation of 440 nm in a 1 cm quartz cuvette with both excitation and emission slit widths at 5 nm. Circular dichroism (CD) spectra were used to characterize conformation changes of human insulin using a JASCO J-810 spectropolarimeter, or equivalent. The spectra were measured using diluted aliquots (to 0.1 mg/mL of insulin) withdrawn at different incubation times from the human insulin or insulin/carbon dot mixture solutions.

ThT is a fibril-specific dye. Thus, insulin fibrillation can be characterized by ThT fluorescence, wherein an increase in fluorescence indicates increased fibril formation. Three stages of insulin fibrillation are observed, which are the lag phase, elongation phase, and saturation phase. The lag phase is the duration during which no fluorescence is observed (i.e., the amount of Tht-fibrils that form, if any, are below the detection limit). The elongation phase is the duration during which an increase in fluorescence is observed as fibrillation occurs. The saturation phase is the duration during which the amount of fluorescence detected levels off as the amount of Tht-fibrils formed saturates the fluorescence signal.

In the absence of carbon dots, 0.2 mg/mL of insulin underwent about 2.5 h of lag phase, followed by 1 h or elongation, and reached saturation after 4 h or incubation. When 0.2 µg/mL of carbon dots were present with the human insulin, the lag phase time of human insulin increased to 3.5 h, about 1 h longer than the insulin sample with no carbon dots present. When the concentration of the carbon dots were increased to 2 and 10 µg/mL, the lag phase of human insulin significantly increased to 5.5 and 12 h, respectively. The CD results were consistent with the observed fluorescence. Human insulin alone at time 0 demonstrated mainly α-helical conformations, and demonstrated β-sheet conformations of mature insulin fibrils after 5 h of incubation, with conformational changes demonstrated between time 0 and time 5 (e.g., shrinking of the peaks indicative of the α-helical conformation and increasing peaks indicative of the β-sheet conformation). A significant increase of the lag time (i.e., time during which α-helical confirmations were observed) was observed for insulin incubated with 2 and 10 µg/mL carbon dots.

Thus, Example 3 demonstrates that carbon dots inhibit human insulin fibrillation and that the inhibition of human insulin fibrillation is carbon dot concentration dependent. The results of Example 3 further suggest that carbon dots of the disclosure stabilize the conformation of human insulin and may address the difficulties in the pharmaceutical industry related to the conformational changes during storage, delivery, and administration of insulin.

### Example 4: Inhibition of Insulin Fibrillation at Lag Phase (reference)

Insulin stock was prepared as in Example 3. The insulin stock solution was used to prepare several samples having insulin concentration of 0.2 mg/mL. To determine the inhibiting effect of 10 µg/mL of carbon dots (prepared according to Example 1) on insulin fibrillation at the lag phase, carbon dots were added after the insulin samples were incubated at 65°C for 0, 1, and 2 h, respectively. Aliquots of samples were taken and checked by ThT fluorescence under the same conditions described in Example 3.

In the absence of carbon dots, the lag time of human insulin was about 2.5 h. When 10 µg/mL of carbon dots were added at time 0, the lag time increased to about 12 h. When the same amount of carbon dots were added to insulin after 1 and 2 h of incubation, the lag time only increased to 5.5 h and 3.5 h, respectively.

Thus, Example 4 shows that carbon dots of the disclosure have a greater inhibiting effect on human insulin fibrillation when added at the earlier stage of nucleation. Without intending to be bound by theory, it is believed that the inhibiting effect is likely due to the interaction between the carbon dots and the insulin species (monomers and oligomers) before the critical nucleation concentration is reached. Once reached, the carbon dots do not change the kinetics of fibrillation. Without intending to be bound by theory, the interaction of the carbon dots with the human insulin species is attributed to weak interactions such as hydrogen bonding, hydrophobic interaction, and van der Waals interactions due to the complicated surface nature of carbon dots. These interactions may be strong enough to adsorb insulin species onto the surfaces of carbon dots, slowing down the self-aggregation and nucleation of human insulin at the early stage of fibrillation. Electrostatic interaction is not expected to contribute much. Due to the protonation of the carboxylic group at pH 1.6, carbon dots do not have much negative charge on their surfaces.

### Example 5: Blood-Brain Barrier Permeability

A zebrafish model was used to test the permeability of the blood-brain barrier to carbon dot-human transferrin conjugates. Zebrafish are a relatively complex vertebrate species with a high degree of physiological and genetic homology to humans. Similar to humans, Zebrafish also possess all major neurotransmitters, hormones, and receptors, including transferrin. The anatomical and physiological conservation in the spinal cord development and function between zebrafish and humans has been demonstrated and proved. Therefore, the zebrafish model enables testing and development of novel therapeutic agents *in vivo.* Another advantage of the zebrafish model is the transparency of the body, allowing the following of pharmacological treatment using non-invasive imaging techniques. Larval zebrafish at 6 d.p.f. with mature BBB were selected as an *in vivo* model.

Carbon dots prepared according to Example 1 were conjugated with one of transferrin, dye-labeled transferrin, or fluorescein (5-(aminomethyl) fluorescein). The dye used to label the transferrin was CF^{™} 594 Dye (Biotium, Hayward, CA). The conjugates were purified by size exclusion chromatography using a size exclusion chromatography column packed from GE Healthcare Sephacryl S-300 (Uppsala, Sweden) or equivalent. UV-Vis absorption was used to confirm conjugation of the transferrin and dye-transferrin to the carbon dots. Transferrin-carbon dots have an absorption around 260 nm and dye-transferrin-carbon dots have an absorption around 594 nm. Circular dichroism spectroscopy was used to determine if conjugation resulted in conformational changes to transferrin or dye-transferrin. No appreciable difference among native transferrin, dye-transferrin, transferrin-carbon dots and dye-transferrin-carbon dots were observed, indicating after conjugation with carbon dots the transferrin and dye-transferrin still maintained the native conformation.

Transferrin was selected to be covalently conjugated to the carbon dots to allow carbon dots to cross the blood-brain barrier via transferrin receptor-mediated endocytosis. It is believed that the transferrin receptor is over-expressed on BBB and the expression of transferrin receptor on the BBB in the larval zebrafish is active at 6 days old. Fluorescein conjugated carbon dots were used to increase the florescence signal of the carbon dots incase the fluorescence intensity of carbon dots was too weak to be seen in the CNS.

Carbon dots or the conjugates were injected intravascularly to the heart of the zebrafish. Confocal fluorescence images were used to detect if the carbon dots or conjugates cross the blood-brain barrier and enter the CNS. Images of control zebrafish without injection demonstrated that the CNS zone is not intrinsically fluorescent.

Un-modified carbon dots did not demonstrate a clear difference from the fluorescence (or lack thereof) demonstrated by the control zebrafish. Fluorescein conjugated carbon dots demonstrated bright fluorescence in the body, but no fluorescence was observed in the CNS. The transferrin-carbon dots were injected under the same conditions as the injection of carbon dots only and no fluorescence difference was observed relative to the carbon dots. Dye-transferrin (transferrin labeled with a fluorescent dye) carbon dot conjugates were injected to the heart of the zebrafish following the same procedures. Fluorescence was observed in the CNS as well as surrounding neuronal cell bodies.

Thus, Example 5 demonstrates that the conjugation system of dye-transferrin carbon dots were able to permeate the blood brain barrier and successfully enter the CNS. It is believed that the transferrin-carbon dot conjugates were also able to permeate the BBB and enter the CNS; however, the intrinsic fluorescence of the carbon dot was too weak to observe.

### Example 6: Bone Binding of Carbon Dots (reference)

Carbon dots were prepared according to Example 1 (denoted C-dots). The surfaces of some of the carbon dots were further modified with one of neutral Biotin (denoted C powder-Biotin), with ethylenediamine to provide positively charged amine groups (denoted C powder-Amine), or with glutamic acid to provide negatively charged carboxyl groups (denoted C powder-Glu). The modifications were done using classical EDC/NHS coupling reactions. Specifically, surface carboxylic groups of the carbon dots were activated by EDC and NHS sequentially, and the activated carbon dots were ten conjugated with the amino moieties on the ethylenediamine and the glutamic acids. Such methods are well known within the art.

Carbon dots were also prepared from glycerol and citric acid (denoted Glycerol and Citric acid, respectively), according to well-known methods as described in "Functionalized carbon dots enable simultaneous bone crack detection and drug deposition." J. Mater. Chem. B 2014, 2, 8626-8632 and "In vitro detection of calcium in bone by modified carbon dots," Analyst 2013, 138, 7107-7111. A sample of the Citric acid carbon dots were surface modified with glutamic acid (denoted Citric acid + Glu).

The carbon dots were introduced into calcified bone of live animals. 5 nanoliters of a solution containing 5 µg/µL carbon dots in neutral phosphate buffer saline were injected into the abdominal cavity of 6 day old zebrafish larvae. Embryos were visualized under the fluorescent microscope 30 minutes after injection. Detection of carbon dots was done using the intrinsic fluorescent properties of the carbon dots. Images were taken in a compound microscope at 100X magnification under bright field transmitted light and fluorescent lights (488 nanometers).

As shown in Figure 1, all carbon dots prepared according to the methods of the disclosure (C powder, C powder-Biotin, C powder-Amine, and C powder-Glu) demonstrated bone binding and, thus, an affinity for calcified bone. In particular, the images demonstrate fluorescence in calcified bone structures in the spinal column (parallel vertical lines) and anterior to the spinal column in the cranial bones, the opercle, and cleithrum. Further, none of the carbon dots prepared according to methods known in the art (Glycerol, Citric acid, or Citric acid-Glu) demonstrated bone binding. In particular, the images demonstrate fluorescence only in detoxifying organs (liver, gut, pronephros). Binding of the carbon dots to bone was confirmed by comparing the fluorescent pattern of the carbon dot images with images prepared from dyes that are known to bind exclusively to calcified bones (e.g., Alizarin Red). It was further found that carbon dots do not bind to tissues that do not have calcium in them. Thus, Example 6 demonstrates that carbon dots prepared according to the methods of the disclosure have a specific affinity for calcified bone not demonstrated by carbon dots prepared by other methods.

### Example 7: Cytotoxicity of Carbon Dots

Gametes were collected from adult sea urchins with ripe gonads. Fresh eggs were washed three times by cold filtered artificial sweater and mixed with sperm to examine fertilization rates. Only eggs with a fertilization rate greater than 95% were used for toxicity tests. 100 healthy fertilized eggs in 2 mL of seawater were deposited in each well of a new, clean, 24-well cell culture plate. Carbon dots prepared according to Example 1 at a concentration of 0, 5, 10, 20, 50, or 100 µg/mL were added to the wells. The plate of fertilized eggs and carbon dots was incubated at 15°C for 16 h until they reached the mesenchyme blastula-stage embryos. Three biological replicates using three individual male-female pairings were then employed. The toxicity of the carbon dots was determined by analyzing the morphology of the embryos after 16 h incubation.

Sea urchin embryos are extremely sensitive to toxic chemicals. The results showed that carbon dots have low cytotoxicity to fertilized sea urchin eggs and embryos. In the presence of 10 µg/mL carbon dots, more than 95% of sea urchin embryos retained a normal morphology after 16 h. Even at high concentrations of carbon dots (i.e., 50 µg/mL carbon dots), more than 90% of embryos remained normal, indicating low cytotoxicity of carbon dots to the cells. The carbon dots were stable in seawater without forming precipitates at concentrations of 50 µg/mL, and at concentrations of 100 µg/mL, precipitates were not observed by the unaided eye after 16 h of incubation, but could be seen with an optical microscope.

Thus, Example 7 demonstrates that carbon dots according to the disclosure demonstrate low cytotoxicity and high stability in sea water.

## Claims

1. A method of forming a blood-brain barrier permeating solution, the method comprising:
covalently conjugating carbon dots with one or more organic compound targets to form carbon dot-organic compound target conjugates and admixing the conjugates with a solvent to form the blood-brain barrier permeating solution, wherein the carbon dots are formed by
admixing carbon nanopowder with sulfuric acid and nitric acid to form a carbon nanopowder mixture, wherein the ratio of sulfuric acid to nitric acid is greater than 1:1 v/v;
heating the carbon nanopowder mixture with reflux to form a refluxed carbon nanopowder mixture and then cooling the refluxed carbon nanopowder mixture;
neutralizing the refluxed carbon nanopowder mixture to form a neutralized carbon nanopowder mixture comprising solubilized carbon dots;
isolating the solubilized carbon dots from the neutralized carbon nanopowder mixture to form a carbon dot solution;
wherein isolating the soluble carbon dots from the neutralized carbon nanopowder mixture comprises crystallizing a salt and removing the salt from the neutralized carbon nanopowder mixture; and
removing impurities from the neutralized carbon nanopowder mixture;
dialyzing the carbon dot solution; and
separating a solvent of the solution from the carbon dot solution to obtain solid carbon dots,
wherein the organic compound target comprises a ligand that is specific to a receptor found at the blood-brain barrier.

2. The method of claim 1, wherein the organic compound target comprises transferrin, dye-labeled transferrin, or any combination thereof.

3. The method of claim 1, wherein the organic compound target comprises amino-containing organic compounds, or alcohol-containing organic compounds, or a combination thereof, and wherein carbodiimide chemistry is used to conjugate said amino-containing organic compounds or alcohol-containing organic compounds to the carbon dots.

4. The method of any one of the preceding claims, wherein heating the carbon nanopowder mixture with reflux comprises heating the carbon nanopowder mixture to 110 °C for 15 hours in a sand bath.

## Patentansprüche

1. Verfahren zur Herstellung einer die Blut-Hirn-Schranke durchdringenden Lösung, wobei das Verfahren Folgendes umfasst:
kovalentes Konjugieren von Kohlenstoffquantenpunkten mit einem oder mehreren organischen Verbindungszielen zum Ausbilden von Konjugaten aus Kohlenstoffquantenpunkten und organischen Verbindungszielen und Vermischen der Konjugate mit einem Lösemittel zum Ausbilden der die Blut-Hirn-Schranke durchdringenden Lösung, wobei die Kohlenstoffquantenpunkte ausgebildet werden durch das Vermischen eines Kohlenstoff-Nanopulvers mit Schwefelsäure und Salpetersäure zum Ausbilden eines Kohlenstoff-Nanopulver-Gemischs, wobei das Verhältnis von Schwefelsäure zu Salpetersäure größer als 1:1 v/v ist;
Erhitzen des Kohlenstoff-Nanopulver-Gemischs unter Rückfluss zum Ausbilden eines refluxierten Kohlenstoff-Nanopulver-Gemischs und dann Abkühlen des refluxierten Kohlenstoff-Nanopulver-Gemischs;
Neutralisieren des refluxierten Kohlenstoff-Nanopulver-Gemischs zum Ausbilden eines neutralisierten Kohlenstoff-Nanopulver-Gemischs, das solubilisierte Kohlenstoffquantenpunkte umfasst;
Isolieren der solubilisierten Kohlenstoffquantenpunkte aus dem neutralisierten Kohlenstoff-Nanopulver-Gemisch zum Ausbilden einer Kohlenstoffquantenpunktlösung;
wobei das Isolieren der löslichen Kohlenstoffquantenpunkte aus dem neutralisierten Kohlenstoff-Nanopulver-Gemisch das Kristallisieren eines Salzes und das Entfernen des Salzes aus dem neutralisierten Kohlenstoff-Nanopulver-Gemisch umfasst; und
Entfernen von Verunreinigungen aus dem neutralisierten Kohlenstoff-Nanopulver-Gemisch;
Dialysieren der Kohlenstoffquantenpunktlösung; und
Separieren eines Lösemittels der Lösung aus der Kohlenstoffquantenpunktlösung zum Erhalten fester Kohlenstoffquantenpunkte,
wobei das organische Verbindungsziel einen Liganden umfasst, der spezifisch für einen Rezeptor ist, der sich an der Blut-Hirn-Schranke findet.

2. Verfahren nach Anspruch 1, wobei das organische Verbindungsziel Transferrin, farbstoffmarkiertes Transferrin oder jegliche Kombination davon umfasst.

3. Verfahren nach Anspruch 1, wobei das organische Verbindungsziel aminohaltige organische Verbindungen oder alkoholhaltige organische Verbindungen oder eine Kombination davon umfasst, und wobei Carbodiimid-Chemie verwendet wird, um die aminohaltigen organischen Verbindungen oder alkoholhaltigen organischen Verbindungen an die Kohlenstoffquantenpunkte zu konjugieren.

4. Verfahren nach einem der vorhergehenden Ansprüche, wobei das Erhitzen des Kohlenstoff-Nanopulver-Gemischs unter Rückfluss das Erhitzen des Kohlenstoff-Nanopulver-Gemischs auf 110 °C für 15 Stunden in einem Sandbad umfasst.

## Revendications

1. Procédé de formation d'une solution perméable à la barrière hémato-encéphalique, le procédé comprenant :
la conjugaison de manière covalente de points quantiques de carbone à une ou plusieurs cibles de composés organiques pour former des conjugués point quantique de carbone-cible de composé organique et le mélange du conjugué avec un solvant pour former la solution perméable à la barrière hémato-encéphalique, dans lequel les points quantiques de carbone sont formés par
mélange d'une nanopoudre de carbone avec de l'acide sulfurique et de l'acide nitrique pour former un mélange de nanopoudre de carbone, le rapport de l'acide sulfurique à l'acide nitrique étant supérieur à 1:1 v/v ;
chauffage du mélange de nanopoudre de carbone par reflux pour former un mélange de nanopoudre de carbone porté à reflux, puis refroidissement du mélange de nanopoudre de carbone porté à reflux ;
neutralisation du mélange de nanopoudre de carbone porté à reflux pour former un mélange de nanopoudre de carbone neutralisé comprenant des points quantiques de carbone solubilisés ;
isolement des points quantiques de carbone solubilisés du mélange de nanopoudre de carbone neutralisé pour former une solution de points quantiques de carbone ;
l'isolement des points quantiques de carbone solubles du mélange de nanopoudre de carbone neutralisé comprenant la cristallisation d'un sel et l'élimination du sel du mélange de nanopoudre de carbone neutralisé ; et
élimination des impuretés du mélange de nanopoudre de carbone neutralisé ;
dialyse de la solution de points quantiques de carbone ; et
séparation d'un solvant de la solution de la solution de points quantiques de carbone pour obtenir des points quantiques de carbone solides,
dans lequel la cible de composé organique comprend un ligand qui est spécifique à un récepteur se trouvant au niveau de la barrière hémato-encéphalique.

2. Procédé selon la revendication 1, dans lequel la cible de composé organique comprend la transferrine, la transferrine marquée par un colorant ou une quelconque combinaison de celles-ci.

3. Procédé selon la revendication 1, dans lequel la cible de composé organique comprend des composés organiques contenant un groupe amino ou des composés organiques contenant un groupe alcoolique ou une combinaison de ceux-ci, et dans lequel la chimie basée sur les carbodiimides est utilisée pour conjuguer lesdits composés organiques contenant un groupe amino ou composés organiques contenant un groupe alcoolique aux points quantiques de carbone.

4. Procédé selon l'une quelconque des revendications précédentes, dans lequel le chauffage du mélange de nanopoudre de carbone par reflux comprend le chauffage du mélange de nanopoudre de carbone à 110 °C pendant 15 heures dans un bain de sable.
